# EUROPEAN PATENT APPLICATION

(11) **EP 3 264 299 A1**
(43) Date of publication of application: **03.01.2018**
(21) Application number: 16176814.8
(22) Date of filing: 29.06.2016
(51) Int. Cl.: G06F 19/00, A01K 5/01, A01K 29/00, A01K 27/00, A01K 5/02

(54) **SYSTEM AND METHOD FOR PET MONITORING**

(71) Applicant: Lovit Labs SAS, 93100 Montreuil (FR)
(72) Inventor: BALMES, Cyrille Stephane, 75011 Paris (FR)
(74) Representative: Witkowska, Weronika

(57) **Abstract**

A system and a method for pet monitoring that enable pet owners, vets or caretakers to remotely monitor and receive information about the health of a pet. The system and method can be useful in monitoring diet results as well as sickness detection. Components of a feeding device (3), along with a portable device(2) worn by the pet (1) and in communication with the feeding device (3), can monitor the pet activity level and type as well as the food and water given (type, calories and nutritional profile). A cloud device (5) may compile and may process collected measurements and perform an analysis based on said measurements and additional information stored in the database (551). The result of the analysis may be used to asses the health and wellbeing of the pet (1) in the light of his caloric, nutritional and energy needs, or issue warnings, notifications related to change of the habits to the owners, vets or caregivers.

## Description

### TECHNICAL FIELD

The present invention relates to a system and method for pet monitoring that enable pet owners, vets or care takers to remotely monitor and receive information about the health of a pet. The system and method can be useful in monitoring diet results as well as sickness detection.

### BACKGROUND

Pets are often considered a family member and maintaining their health and longevity is a constant need for their owners,

Document EP2713709 discloses a method and a system for monitoring the behavior of an animal. The system comprises a sensor configured to detect the presence of the animal, a measuring device configured to measure a characteristic of the animal whenever presence of the animal is detected; and a storage device configured to store the measured characteristics of the animal. Moreover the system comprises a processor configured to calculate derived data from the measured characteristics, the processor being remote cloud device. The method comprises detecting the presence of the animal, measuring a characteristic of the animal whenever presence of the animal is detected and storing the measured characteristics of the animal. Moreover the method comprises calculating derived data from the measured characteristics.

However said known system gives only the possibility to monitor the environment of the pet and its behavior like it is or monitor deviations from the typical characteristics. It does not allow the pet owner to get the information on how to adjust intentionally and properly different factors influencing the wellness of the pet so the wellness of the pet becomes better.

In general all known systems do not take seriously into account the fact that owners vary the food (use more than one food source) almost everyday for most of them (70%) and do not control it. The quantity of food given is often based on judgment and owners do not take into consideration the pet lifestage (kitten, adult, senior), size (small bread vs. large bread) and true level of activity.

While, research conduct by Nestle Purina (Canine Lifespan diet restriction study) have shown that Pets who keep lean body condition and received food matching their energy requirement can live up to 2 years longer, research conduct at WALTHAM Centre for Pet Nutrition (Macronutrient Selection study) have shown additionally that Pets will naturally seek a diet matching a specific macronutrient profile for optimal health. It is therefore not only the quantity eaten but as well the nutritional composition of the food given that can ensure health and longevity for the pets.

Pet feeding dishes or dispensers are commercially available; However, those feeding dishes or dispensers typically only control the amount of food given to the pet via owners' predefined setting and do not take into account the actual level of energy requirements and are additionally limited to one type of food only and excluding for the vast majority the provision of water. Furthermore, those known dishes or dispensers typically do not guarantee easy monitoring of multiple pets.

Thus the technical problem to be solved is to provide a system and method that overcome the aforementioned drawbacks, namely which guarantee more detailed and reliable monitoring of pet's health by taking into account simultaneously various additional factors and allows the pet owner to adjust some factors so the pet is of optimal health, while enabling monitoring of multiple pets.

### SUMMARY

According to the first aspect, the object of the invention is to provide a system for pet monitoring comprising:
- a portable device 2 comprising a data storage for storing at least an individual pet identifier, an activity and motion sensor and a communication module for sending collected measurements and for informing about the proximity of said individual pet 1;
- a feeding device 3 comprising a data storage module, a weight sensor module, at least one feeding and/or drinking bowl, and/or a first communication module and second communication module;
- means for food and/or drink type recognition;
- a cloud device 5 comprising a database, remotely located relative to the feeding device and portable device, for receiving and analyzing at least consumption measurements and the measurements indicative of at least type and amount of the food and/or drink consumed, nutrition measurements including product nutrition information by the pet transmitted by the feeding device;

Advantageously, means for food and/or drink type recognition are part of the feeding device.

Advantageously the system further comprises at least one pet owner terminal device 7 configured to communicate with the cloud device for receiving at least a portion of the processed measurements and result of analysis as well as recommendation messages.

Advantageously, means for food and/or drink type recognition are part of the terminal.

Advantageously, the portable device comprises means for measuring physiological data.

Advantageously the system further comprises a weight device 4 for measuring at least the weight of an individual pet.

Advantageously, the cloud device is configured to process collected measurements and/or performs data analysis and generates recommendation messages based on at least activity and motion measurements as well as consumption measurements and nutrition measurements.

Advantageously, the cloud device comprises a webserver hosting a website for providing the pet owner 6 through a terminal device 7 with at least a portion of the processed measurements and result of analysis as well as messages.

More advantageously, the database stores at least one among the following: scientific information pet's data related to the major pet medical conditions data and their impact on the pet weight, typical breed's and specie's physiological characteristics per life stage such as typical breed's and specie's weight, typical breed's and specie's level of activity, typical breed's and specie's food consumptions and typical breed's and specie's energy requirements.

According to another aspect, the object of the invention is to provide a method of pet monitoring comprising:
- A step of providing the cloud device with measurements data being at least activity and motion measurements, consumption measurements including the amount of food eaten, the amount of water drank and nutrition measurements including product nutrition information;
- A step of performing analysis of at least activity and motion measurements, feeding measurements including the amount of food eaten, the amount of water drank and nutrition measurements including product nutrition information so as to determine output pet characteristics including feeding pattern 501, activity/behavior pattern and health/ wellness evaluation pattern including at least individual level of energy requirement;
- A step of matching current output pet characteristics with output pet characteristics saved in database, so as to determine change and/or deviation in pet characteristics;

Advantageously, the method further comprises a step of generating messages to the owner/vet/care taker 6 and transmitting it to the pet owner terminal device 7.

Advantageously, the method further comprises a step of providing a cloud device 5 with pet's data comprising at least one among species, breed, age, sex, weight, nurtured state, medical history, DNA profile, date of birth and/or scientific pet's data comprising at least one among the major pet medical conditions data and their impact on the pet weight, typical breed's and specie's physiological characteristics per life stage such as typical breed's and specie's weight, typical breed's and specie's level of activity, typical breed's and specie's food consumptions, typical breed's and specie's energy requirements and storing in the cloud database.

Advantageously, weight measurements like the weight of the pet, the weight of the pet excrements are provided in the step of providing the cloud device with measurements data.

The system including several devices along with a terminal device used by a pet owner enables the pet owner to remotely monitor and received information about the individual nutritional profile (macro and/or micro nutrient) and/or energy requirement and food/calories and/or water consumption of his pets (no matter if the pet owner is feeding the pet with different type of food, has more than one pet and cannot always observe the level of his pet activity).

In particular, components of a feeding device, along with a portable device worn by the pet and being in communication with the feeding device, can identify the pet and monitor the pet activity level as well as the food and water given (type, calories and nutritional profile).

A cloud database may compile and may perform an analysis of amount of calories eaten and/or of water drank and measured nutritional profile of the food sent from the feeding device Further it may combine that data with analysis of pet activity level that may be translated into energy requirements of individual pet. The result of the analysis may be used to assess the health and wellbeing of the pet in the light of his caloric, nutritional and energy needs, and/or to issue warnings, notifications related to change of the habits to the owners, vets or caregivers and indications what environmental factors should be changed.

Thanks to the structure of the portable device which is adapted to store an original identifier (a pet unique identification code) for a specific pet among multiple pets the portable device as well as other devices in the system are adapted to record separately for each pet different characteristics, for example the amount of food and water eaten by a specific pet, therefore the system remedies the problem to deliver the pet with tailored nutrition adapted to his energy requirement when the owner decide to mix different commercial food products and/or have more than one pet.

An advantage of the present disclosure is to monitor the activity of a pet, obtain real-time pet health and wellness data and use this information to provide the pet owner with the individual energy requirement of his pet.

Another advantage of the present disclosure is to monitor not only the amount eaten but as well what has been eaten and therefore use the information to provide the pet owner with his pet's current nutritional profile and difference between his pet's tailor nutritional requirement based on scientific feeding rules, the food nutritional composition, the level of activity and the past data about the pet and/or in comparison to other pets with similar characteristic.

Another advantage of the present disclosure is to monitor not only the amount eaten but as well the food type and real calories eaten by the pet and therefore use the information to provide pet owner with difference between his pet's calorie consumption and scientific feeding rules regarding the lifestage (kitten/puppy, adult, senior, super senior) or size of the pet.

Another advantage of the present disclosure is to identify individually each pet using the devices and therefore enable the owner to serve multiple pets with one single feeding device.

Another advantage of the present disclosure is to allow the pet owner to mix different type of food (brands, flavors, formats, texture...) into multiple or one single feeding device as well as to match the existing owner-feeding pattern. Thanks to the combination of the weight sensor module and means for food and/or drink type recognition it is possible for the pet owner 6 to freely mix within on meal and/or across meal and/or within one feeding bowl both the quantity and type of food given to the pet while obtaining accurate real-time measurements regarding the pet's consumption and nutrition measurements.

Another advantage of this solution is to use scientific knowledge to remotely predict the pet activity, health and individual calorie and nutritional requirement and current consumption and help alert the owner of any sudden change.

Yet another advantage of the present disclosure is to locally collect data regarding pet activity and health conditions using sensors; send the data from sensors to the feeding device; and then send the data from the feeding device to a cloud database and/or a remotely located terminal device.

Yet another advantage of the present disclosure is the ability of the feeding device to locally store the measurement data and therefore avoid the need of permanent connection to the Internet.

Still another advantage of the present disclosure is to form a consumer-club of pets and enable each owner to support each other's.

Yet another advantage of the present disclosure is to maintain an history of individual health and wellness data, an activity profile and an eating profile of each pet as well as more specific data such as the level of activity, the feeding/nutrition consumption.

Yet another advantage of the present disclosure is to make the information/data automatically available to a vet or care taker at the discretion of the owner of the pet.

Still another advantage of the present disclosure is to track the evolution of the pet with regard to a specific feeding program such as a weight reduction program to achieve health and wellness.

Still another advantage of the present disclosure is to use not only macro information, namely pet's data input by the owner such as the pet age, breed and sex but also a combination of precise micro information such as the pet genetic, the activity/motion measurement and the real-time food consumption and nutrition data in order to define the pet tailored nutritional and energy requirements.

### BRIEF DESCRIPTION OF DRAWINGS

Additional features and advantages are described in, and will be apparent from, the following detailed description and the figures:
- Figure 1 a is a schematic diagram of an embodiment of a pet monitoring and feeding system according to the invention.
- Figure 1b is a schematic diagram of another embodiment of a pet monitoring and feeding system according to the invention.
- Figure 2 is a more detailed block diagram of an embodiment of the system of the pet monitoring and feeding system according to the invention.
- Figure 3 is a schematic diagram of an embodiment of the feeding device of the pet provided by the present disclosure.
- Figure 4 illustrates an embodiment of a cloud database analytic engine provided by the present disclosure.
- Figure 5a and 5b illustrate an embodiment of a web interface hosted by the cloud device according to the invention.
- Figure 6 illustrates steps of the method according to the invention.

### DETAILED DESCRIPTION

The term "pet" means a dog or a cat.

The term "pet owner" is an enlarge definition of the actual owner of the animal and can include his vet or any person looking after his care.

The term "pet food" and "food" mean any composition that may be consumed by a pet. In an embodiment, the pet food may be water.

The term "nutrition" means the process of taking and/or providing in food and using it for growth, metabolism, and repair. The commonly accepted nutritional stages are ingestion, digestion, absorption, transport, assimilation, and excretion.

The term "tailored and/or individual nutrition" means the process of adjusting the nutrition provided for a particular purpose, situation, or need.

The term "nutritional profile and/or composition" refers to the nutrient composition of a food or diet. It includes the macronutrients such as water, protein, carbohydrate and fat and/or breakdown in sub elements (for example, the nutritional profile can include the carbohydrate amount and/or the break down of the carbohydrates in sub categories such as sugars, starch and fibers), the micronutrients (for example, the nutritional profile can include the amount of amino acids and/or fatty acids), the vitamins and/or the minerals.

The term "energy requirements" means the amount of dietary energy an animal needs to meet its energy requirement accounting for but not exclusively:
- The basal metabolic rate (energy expended to maintain basic physiological functions including heart beat, respiration and muscle function which is usually measured as resting metabolic rate.)
- The thermogenesis (energy expended to ingest, digest, absorb and assimilate food as well as to cope with stress, including changes in environmental temperature.)
- The activity (energy expended during movement and exercise.)

The term "cloud device" means the hardware (e.g., a computer) and/or the software (e.g., a computer application) that receives, stores, processes and delivers content that can be accessed through the internet, for example, using a website hosted by the cloud device and/or a web server associated with the cloud database.

As used herein and in the appended claims, the singular form of a word includes the plural, and vice versa, unless the context clearly dictates otherwise. Thus, the references "a," "an" and "the" are generally inclusive of the plurals of the respective terms. For example, reference to "a measuring device" or "a method" includes a plurality of such "measuring devices" or "methods." Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" also is a disclosure of embodiments "consisting essentially of" and "consisting of" the disclosed components. Where used herein, the term "examples," particularly when followed by a listing of terms, is merely exemplary and illustrative, and should not be deemed to be exclusive or comprehensive.

The terms "remotely" and "locally" are in reference to the environment in which the device is located. Preferably "locally" refers to a domestic or in-home environment, but the present disclosure is not limited to a specific location in which the device is located.

The term "automatically" means without user input being necessary. An "automatically" performed operation can comprise one or more actions by the corresponding device, but each of the actions is performed without a requirement of user input.

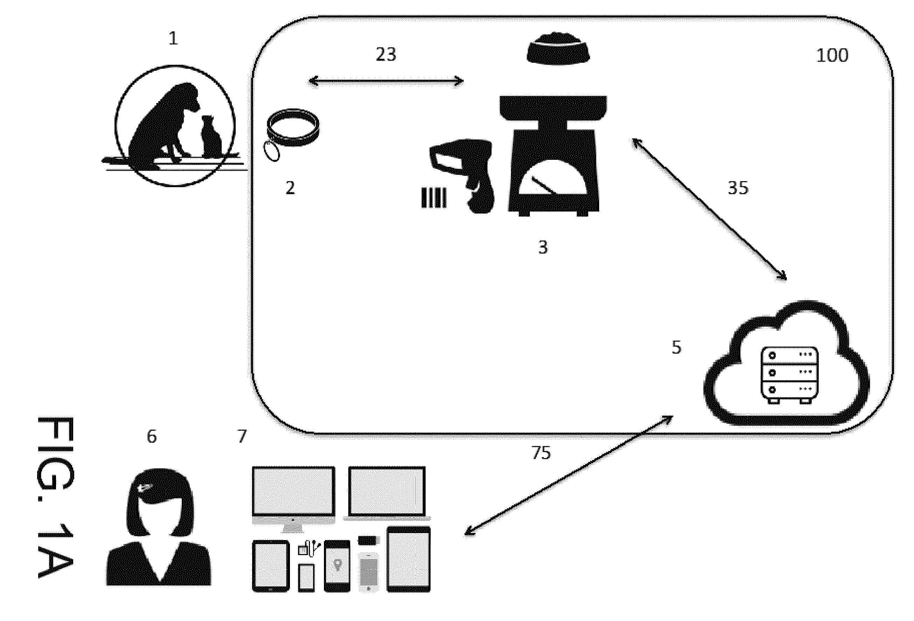

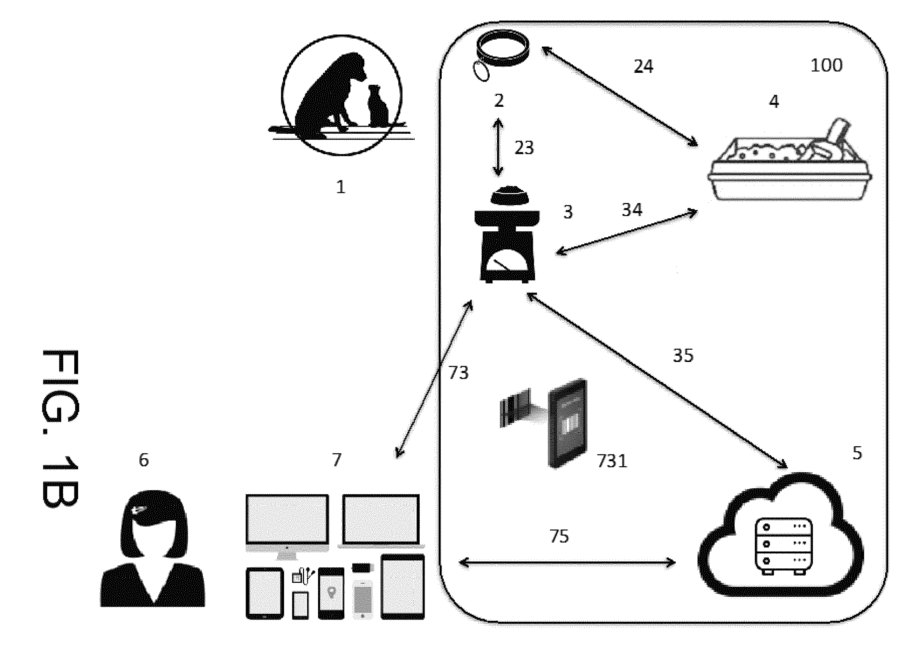

An embodiment of the system for the pet monitoring according to the present invention is illustrated in Figure 1a and Figure 1b. The system according to the invention is a remote pet monitoring and recording system 100 that enables a pet owner 6 to monitor, record information and feed a pet 1. The system 100 comprises a feeding device 3, a cloud device 5 and a portable device 2 (tracking device) that is worn by the pet 1. The cloud device 5 communicates with a terminal device 7 of the pet owner.

For example, the portable device 2 may be a collar worn by the pet 1 and/or can be configured to be attached to an existing collar worn by the pet 1.

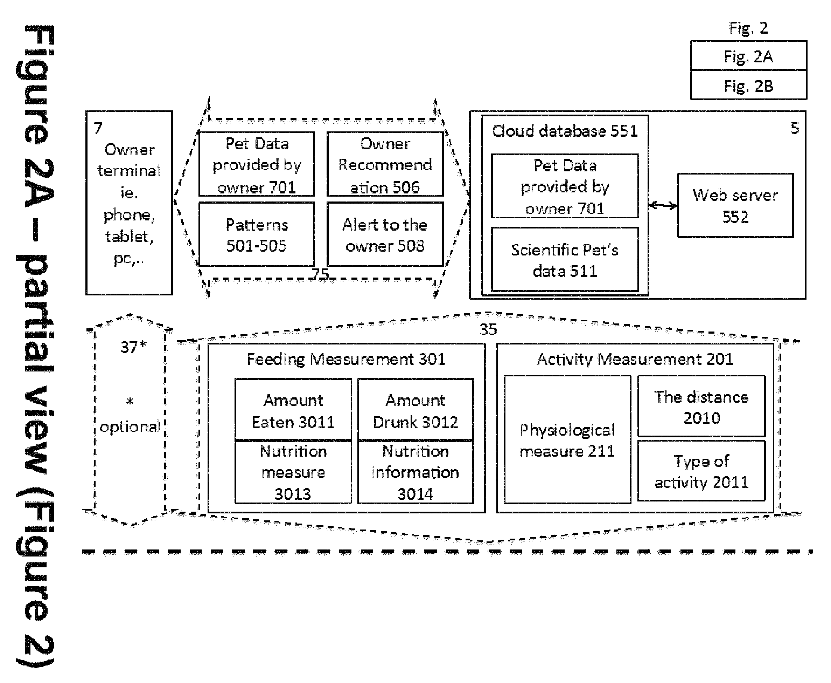

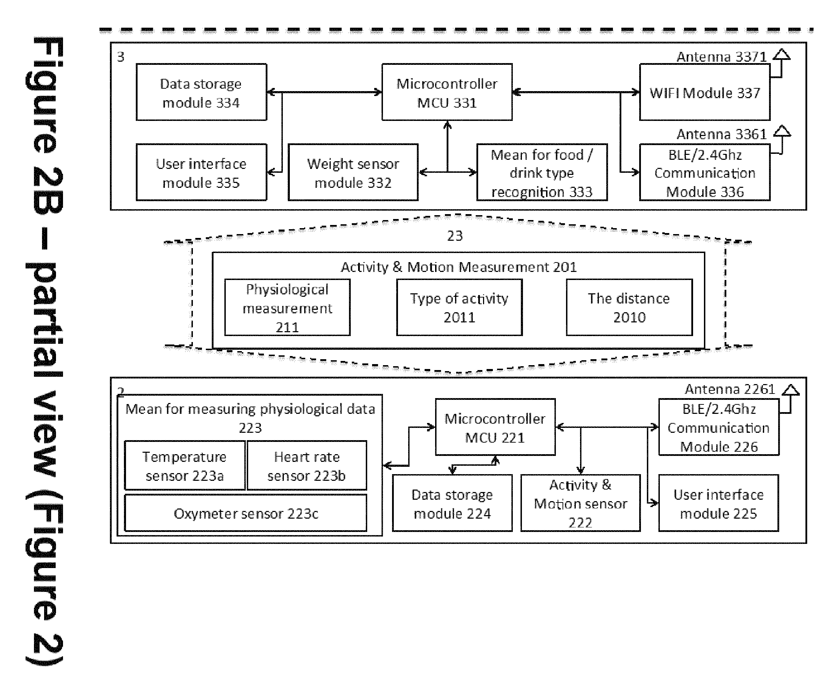

The portable device 2 can automatically acquire real-time activity and motion measurements 201 regarding the pet 1. As shown in figure 2, for example, an activity and motion sensor 222 integrated in the portable device can measure the activity and motion spent during a time period, the distance 2010 moved by the pet 1 during a time period as well as the type of activity 2011 performed by the pet during a time period. In addition the portable device 2 can and/or may comprise also means 223 for measuring physiological data regarding the pet 1, for example real time heartrate 2110, temperature 2111, blood oxygen level 2112. The portable device 2 can comprise at least one of following: an activity and motion sensor 222, a temperature sensor 223a, a pulse oximeter 223b or a heart rate monitor 223c as well as a wireless communication module 226. The portable device 2 is able to collect pet real time level of activity of the pet 1 as well as other real time physiological characteristic of the pet as needed through the adjunction of additional sensors. The activity and motion measurements 201 comprise the acceleration characteristic features (statistical moments) after having compensating the data for the effect of gravity.

In one embodiment the portable device 2 comprises a communication module 226 which can communicate at least with the feeding device 3 using a wireless connection 23 and transmit the measurements collected regarding the pet 1 to the feeding device 3 in order to store it contemporary. The person skilled in the art will know that transmission can be realized also thanks to a relative proximity of the portable device 2 and the feeding device 3.

Details of the portable device 2 are illustrated in Figure 2. As shown in Figure 2 the portable device 2 comprises an microcontroller 221 responsible for operating the device, a data storage module 224 responsible for storing all measurements performed by the portable device 2, an activity and motion sensor 222, means 223 for measuring physiological data, for example a temperature sensor, a user interface module 225 and a communication module 226 which is a BLE/RFID/NFC radio module 226 and its antenna 2261. In the present embodiment, the portable device 2 comprises a power source 228 (not shown) like removable battery with extended life to ensure the portable device 2 will be worn by the pet 1 as long as possible without any need of the owner to touch it. The present disclosure is however not limited to a specific form of power source 228 but must allow the device 2 to be worn by the pet 1 for extended period of time without altering the pet's mobility in any form or way. By sending an identifier the communication module 226 informs all other devices of the system 100 about the proximity of a certain pet among multiple pets if a certain number of pets is present at home. The pet owner enters the identifier of a certain pet via a user interface module 225 which is stored in a data storage module 224 during the registration/device setup process.

In an embodiment, the portable device 2 can comprise as means 223 for measuring physiological data at least one among: a heart rate monitor 223b, a pulse oximeter 223c, a glucose meter 223d and/or any physiological sensor available in order to obtain further physiological measurements 211 of the pet 1.

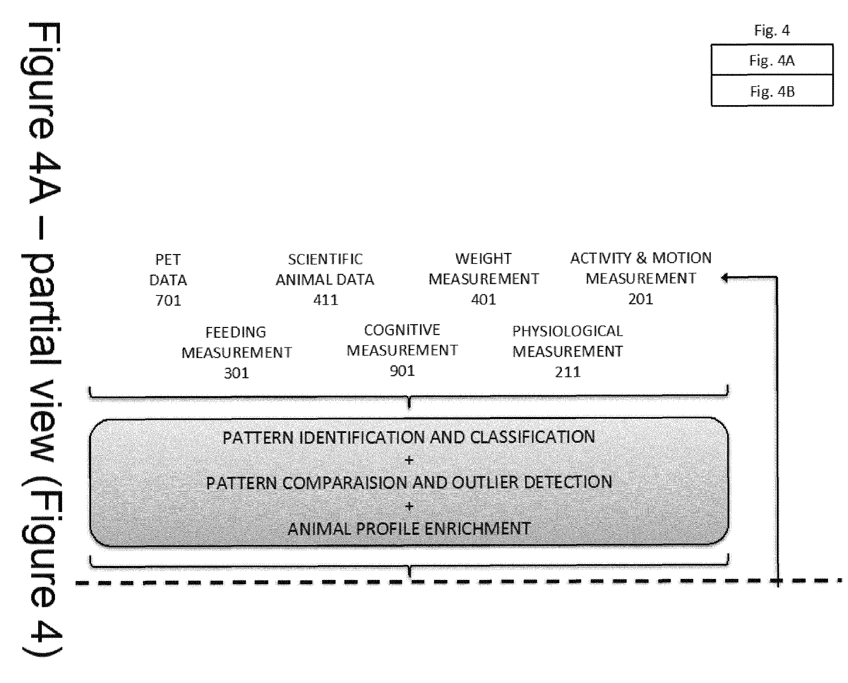

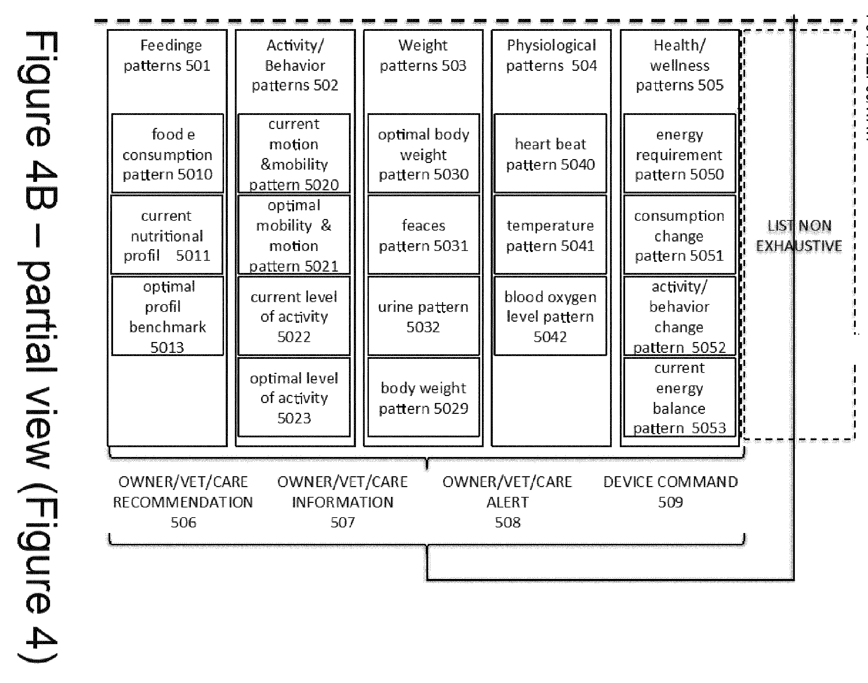

The system according to the present disclosure will be able to identify the social interaction between two pets wearing a portable device 2 by detecting the proximity of said two pets as well as define specific activity/behavior patterns 502 (see figure 4). In addition the system according to the present may also include a smart toy 9 (not shown) that could stimulate and interact with a pet wearing the portable device 2 once the toy 9 has detected the proximity of the given pet 1. In consequence, play/cognitive measurements 901 can be acquired and sent to the cloud device 551.

As shown in Figure 2, the feeding device 3 comprises a weight sensor module 332, for example a scale 332 where the owner 6 can place a feeding bowl(s) 339 holding pet food, means for food and/or drink type recognition 333, for example an optical scanner 333, and a first communication module 336 and/or a second communication module 337. The owner 6 is free to use any feeding bowl 339 available on the market as long as it could fit on the scale 332 surface to ensure proper weight measurement of the food, namely to receive a proper amount of food eaten 3011 and amount of water drank 3012. The scale 332 can automatically calibrate itself to the new feeding bowl 339. As discussed in detail hereafter, the feeding device 3 acquire feeding measurements 301 and can not only measure with precision the amount eaten by the pet 1 using the scale 332 but also the type of food eaten and its nutritional composition, namely acquires nutrition measurements 3013 including product nutrition information 3014 using for example additional means for food and/or drink type recognition 333, for example an optical scanner 333 as well as the pet 1 unique identifier using the communication module 336. The person skilled in the art will appreciate that the pet's unique identifier can be read out using RFID/NFC technology.

As shown further in Figure 2, the feeding device 3 comprises a microcontroller 331 responsible to operate the device, a data storage module 334 responsible to store all measurements performed by the feeding device 3, a weight sensor module 332, means for food and/or drink type recognition 333, a user interface module 335, a second communication module 337, for example a WIFI module 337 and its antenna 3371 and a first communication module 336, for example BLE/RFID/NFC radio module 336 and its antenna 3361.

In another embodiment, a mobile phone equipped with a camera as well as a dedicated application software can replace the feeding device's optical scanner module 333. In another embodiment, a keypad and a screen coupled with a predictive and intuitive algorithm can replace the feeding device's optical scanner module 333, which means that means for food and/or drink type recognition 333 might be also a module separate from the feeding device 3.

The weight sensor module 332 can measure accurately the amount of food and water put into the feeding bowl 339 by the pet owner 6 as a part of the pet's 1 consumption measurements 3010; The optical scanner module 333 can measure/scan the food package given by the pet owner 7 and determine therefore the nutrition measurements 3013 of each food put into the feeding bowl 339.

As already mentioned, means for food and/or drink type recognition 333, for example the optical scanner module 333 can be located locally from the feeding device 3 as illustrated in Figure 1a or remotely from the feeding device 3 as illustrated in the Figure 2 with a scanning software 771. The present disclosure does not limit the form of means for food and/or drink type recognition 333 but means for food and/or drink type recognition 333 must discriminate and recognize accurately the different type of food put into the feeding bowl 339 by the pet owner 6.

The combination of the weight sensor module 332 and means for food and/or drink type recognition 333 can allow the pet owner 6 to freely mix within on meal and/or across meal and/or within one feeding bowl 339 both the quantity and type of food given to the pet 1 while obtaining accurate real-time feeding measurements 301 regarding the pet's consumption measurements 3010 and nutrition measurements 3013.

The feeding device 3 can therefore automatically acquire individual and real-time consumption 3010 and nutrition measurements 3013 over the time period regarding each pet 1.

In the present disclosure, the consumption measurements 3010 comprise times that the pet food and water was consumed, the pet unique identification code and, for each of the times, an amount of food and/or water consumed. The nutrition measurements 3013 comprise times that the pet food was added to the feeding bowl and, for each of the times, product nutrition information 3014, for example the product unique identification code in the form of an EAN code and any other form of unique product identification (eg. calories per gram, fat, protein, carbs, water content, micronutrients content). The feeding device 3 can operate in standard mode where the owner 6 will need to scan the package of every food given to the pet 1 or in predictive mode allowing the owner 6 to only scan the food package when a change of food occurs.

As shown in figure 2, the second communication module 337 integrated in the feeding device 3 can use a wireless connection 35 to automatically transmit the real-time consumption 3010 and nutrition measurements 3013 collected as well as real-time activity and motion measurements 201 received from the portable device 2 to a cloud device 5. For example the feeding device 3 can transmit the measurements directly after receiving the data from the portable device 2 and/or perform the task at predetermined time intervals. The wireless connection 35 is bi-directional allowing the feeding device 3 to know if the measurements have been safely sorted in the cloud device 5 as well as to receive software update.

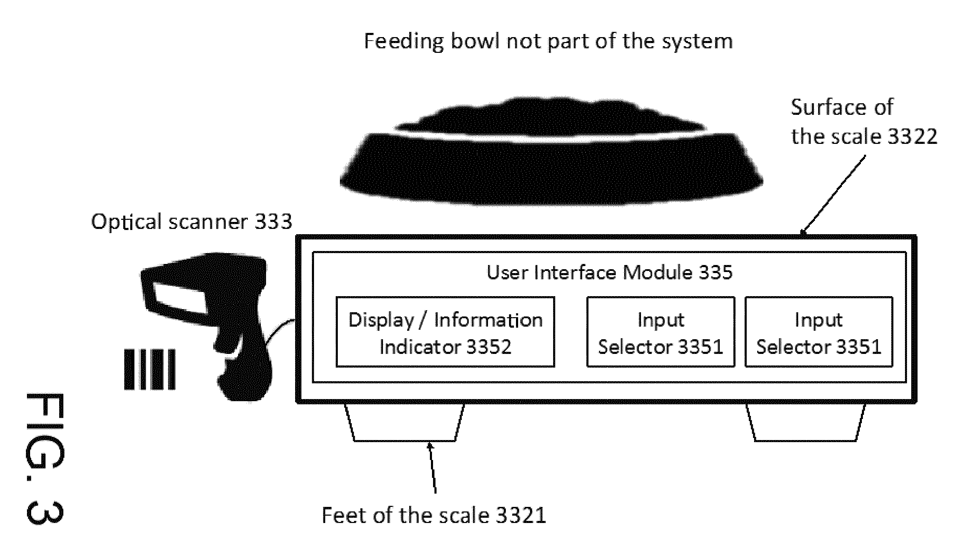

The feeding device is also shown in Figure 3. The user interface module 335 comprises one or more input selectors 3351 and information indicators 3352 to notify, act, guide or inform the pet owner 1, for example on different actions already done or to be done. The present disclosure is not limited to a specific user interface module. It can be any device that can display information to the pet owner 6 and/or allow him to provide input to the system, for example a combination of buttons and led status indicator can be used but as well a tactile screen or a buffer.

As shown in figure 2, furthermore, the feeding device 3 can also communicate with the terminal device 7 of the pet owner bi-directionally, either directly using one or more wireless connections or indirectly using the cloud device 5. The second communication module 337, namely the WIFI module 337 and its antenna 3371 can transmit the device's measurements to the cloud device 5 and receive information from it. In one embodiment, the feeding device can connect to Internet via the pet owner mobile phone.

The first communication module, namely the BLE/RFID/NFC radio module 336 of the feeding device 3 and its antenna 3361 can transmit measurements, information, commands to the portable device 2 and can also measure the proximity of the pet 1 in relation to the device as well as identify the pet. The present disclosure is not limited to a specific transmission and can be any data that can be exchange between the feeding device 3 and the portable device 2.

As already mentioned, figure 2 schematically illustrates an embodiment of the system 100 as shown in Figure 1 a. The cloud device 5 comprises a database 551 and a web server 552 that can communicate bi-directionally with the system 100 using the second communication module 337, i.e. WIFI module 337 embedded in the feeding device 3 as well as with the pet owner terminal device 7.

The cloud database 551 stores at least the following information: pet's data 701 provided by the owner like species 7010, breed 7011, age 7012, sex 7013, weight 7014, nurtured state 7015, medical history 7016, DNA profile 7017,date of birth 7018 and scientific pet's data 511 comprising at least one among the major pet medical conditions data 5110 and their impact on the pet weight, typical breed's and specie's physiological characteristics per life stage such as typical breed's and specie's weight 5111, typical breed's ,specie's level of activity 5112, typical breed's and specie's food consumptions 5113 , typical breed's and specie's energy requirements 5114. In the present embodiment, the information stored in the cloud database is also previously compiled data for the pet, previously compiled data for other pets that are the same type of pet, and a combination thereof. The same type of pet is determined at least partially using a criterion selected from the group consisting of pet species, pet breed, pet age, pet weight, and combinations thereof. In a related embodiment, the same type of pet is determined at least partially using an extract from the criterion describe above as well as the pet individual genomic sequence.

The terminal device 7 can be any device having the communication module 736 (not shown) that can access the Internet and comprises display means for displaying a website and do not limit the present disclosure. For example, the terminal device 7 can be at least one of a mobile telephone, such as a smartphone, a laptop or desktop personal computer or a tablet.

During the installation of the system, the cloud database 551 is able to collect pet's data 701 such as species 7010, breed 7011, age 7012, sex 7013, weight 7014 nurtured state 7015, medical history 7016, DNA profile 7017, date of birth 7018 through the use of the terminal device 7 and entered by the pet owner. Scientific pet's data 511 can be acquired from some other sources like other external databases.

As illustrated in figure 4 in details, the cloud device 5 can collect different measurements data and then perform analysis using said nutrition measurements 3013, consumption measurements 3010, activity and motion measurements 201 of the pet 1 both present and in the past as well as stored information regarding detail product nutrition and/or scientific information.

Each measurement collected by the system will be processed into pattern and producing by not exclusively the following patterns output:
- Feeding patterns 501
- Activity & Behavior patterns 502
- Weight patterns 503
- Physiological patterns 504
- Health/ wellness evaluation patterns 505

In this regard the cloud device 5 performs pattern identification, which is processing of single input data and measurements in order to create patterns. This step can for example include the filtering and/or sampling and/or the computation of statistical features of the input data such as the mean, the variance, the skewness, the kurtosis and/or the covariance of a given lag.

More over the cloud device 5 performs pattern classification, which is processing of a sequence of single pattern type in order to create meta representation of the pet 1. A typical pattern classification could involve the activity and motion measurements 201 in order to classify the pet motion into recognizable movement such as sleeping, walking, playing, jumping, purring.

For example, the cloud device 5 will be able to use the pet's data 701 such as breed 7011, age 7012, sex 7013, weight 7014 nurtured state 7015 and activity and motion measurements 201 acquired through the portable device 2 to define like health/ wellness evaluation pattern 505, for example the individual level of energy requirement 5050 for the pet 1. Combining the information collected by the feeding device 3, the cloud device 5 will then be able to evaluate through the identification of the amount eaten and type of food eaten (consumption measurements 3010), the food consumption pattern (frequency, type and calorie) 5010 and current nutritional profile 5012 of the pet 1.

The cloud device 5 also performs pattern comparison, which is processing of the classified patterns and comparison of those patterns against reference patterns in order to highlight deviation, which will trigger information and/or recommendation.

For example, matching one of the health/ wellness evaluation pattern 505 like individual level of energy requirement 5050 and the food consumption pattern (frequency, type and calorie) 5010, the cloud device 5 will then be able to compare the balance of both to the optimal needs of the pet as well as predict trends and consequences which may impact the future of the pet 1 as well as provide recommendations 506 to the pet owner 6 regarding any changes in feeding behavior.

Matching the current nutritional profile 5012 of the food eaten with optimal nutritional profile 5013 for the pet 1, the cloud device 5 will be able to compare the balance of both and predict trends and consequences which may impact the future of the pet as well as provide recommendations 506 to the pet owner 6 regarding any changes in feeding behavior.

For example the cloud device 5 will pattern classification activity and motion measurements 201 of the pet 1 to identify early sign of illness such as worm or parasite infection by not only recognizing the change of behavior of the pet 1 but as well identifying the key features of the behavior change and matching those features to scientific pet's data 511 present in the cloud device 5.

The owner 6 can use his terminal device 7 to connect to the cloud device 5 and review and/or receive the result of the analysis performed on the nutrition 3013, consumption 3012, activity and motion measurements 201. For example, the owner (6) can review the food consumption pattern (frequency, type and calorie) 5010 of the pet 1 (ie. when is the pet 1 eating and how much quantity), the current nutritional profile 5012 of the food eaten by the pet 1 as well as the comparison to the optimal profile 5013 for the pet 1. The owner can also review the current motion and mobility pattern 5020 (i.e when is the pet 1 playing/moving and how much).

The cloud device can send different types of messages to the owner/vet/care taker. For example it might be a recommendation, which informs the owner/vet regarding a quick actionable step the owner/vet can take, can do in order to improve the wellbeing of the pet. The cloud device according to the invention can send a message including a feeding recommendation (506) such as changing the type of food given and/or changing the water more frequently. This can also include a cleaning recommendation (507) such as cleaning the litter box at more regular occasion. The recommendation can also include a full detail report highlighting the major facts that have lead to the recommendation the owner/vet can review.

The cloud device 5 can also send an alert 546 to the owner 6 once a change in the food consumption pattern (frequency, type and calorie) 5010, nutritional profile 5012, Activity/behavior pattern 502 is observed. Moreover the cloud device 5 performs pattern outlier detection, which is processing of the historical classified patterns and recent pattern comparison to identify false positive case before triggering an alert.

The cloud device 5 can use SMS, email or information displayed on the cloud device 5 webserver via special interface 550 or any mean of communication that is best appropriate to inform the owner 6 about the health and wellness state of the pet 1.

In another embodiment illustrated in Figure 1b, the system 100 can also comprise a weight device 4 that is placed under an area where the pet 1 would rest for some time during the day and over the time. For example, the weight device 4 can be placed under the pet bed and/or be placed under the litter box 8 of the Pet 1 so as to ensure the nutrition cycle is closed

The weight device 4 can automatically obtain real-time weight measurements 401 regarding the pet 1. For example, it can measure the weight of the pet 4010 during different time periods. Moreover it can measure the weight of the pet excrements 4011 (both liquid and/or solid) generated by the pet 1 during a certain time period when the device is placed under the litter 8 of the pet 1. In addition the weight device 4 can also comprise means (413,414) for measuring the real-time humidity and air quality as part of obtaining environmental measurements 402 regarding the pet 1 (not shown). In this regard, the weight device 4 can comprise at least one of following: scale sensor 411, a temperature sensor, 412, a humidity monitor 413 an air quality sensor 414. The weight device 4 also comprise a communication module 436 which can communicate with the feeding device 3 using a wireless connection 34 as well as with the portable device 2 using a wireless connection 24. It is adapted to transmit the measurements collected regarding the pet 1 to/or from the feeding device 3 and portable device 2 as well as an information on its relative proximity to the other devices leveraging the Radio Signal Strength Index (RSSI) provided by the radio communication module 436 to be used in triangulation algorithms.

In this embodiment illustrated in Figure 1b, the BLE/RFID/NFC radio module (336) of the feeding device 3 and its antenna 3361 can communicate with the weight device 4 as well as the pet owner 6 terminal device 7.

Moreover in this another embodiment the schematic of the weight device 4 can mirror the schematic of the feeding device 3 shown in Figure 2 and the optical scanner 333 can be replaced or enriched by a humidity sensor 413 and/or pH sensor 414 to obtain additional physiological measurements 403 of the pet 1. The functionality and role of the BLE/RFID/NFC will be similar as the one present in the feeding device 3 i.e. receiving data regarding the pet 1 identification and proximity as well as mean of communication between the device and/or terminal device 7 of the pet owner 6.

In this embodiment, the cloud device 5 can use weight measurement 401 provided by the weight device 4 to evaluate and predict health/wellness evaluation pattern 505 cause by the current feeding imposed by the owner 6. In addition, by measuring the weight of the pet excrements 4011 (both liquid and/or solid) produced by the pet 1, the cloud device 5 can predict additional sign of illness.

In another embodiment, the Internet connection present in the feeding device 3 can be replaced by an Internet connection present in the weight device 4. In this instance, the weight device 4 will become the gateway to transfer the measurement data, the portable 2 and feeding device 3 and all communication methods describe above for the feeding device 3 apply to the weight device 4.

In one embodiment, when the system include an additional weight device 4 placed under a litter box 8, the weight measurements 401 will also comprise times that the pet was located on the device 4, the pet unique identification code (identifier) and, for each of the times, the weight of the pet excrements 4011.

In another embodiment of the system 100 illustrated in Figure 1b, the terminal device 7 can communicate bi-directionally with the system 100 and can have the possibility to install the scanning software 731 described previously. The scanning software 731 can obtain real-time nutrition measurements 3013 like product nutrition information 3014 (type of the food given to the pet) and therefore can replace the need of means for food and/or drink type recognition 333 in the feeding device 3 (i.e the optical scanner 333). Additionally, the scanning software 731 being implemented on the owner terminal 7, can transfer real-time nutrition measurements 3013 through the communication module 736 to the feeding device 3, the last one forwarding all or part of the collected real-time measurements to the cloud device 5.

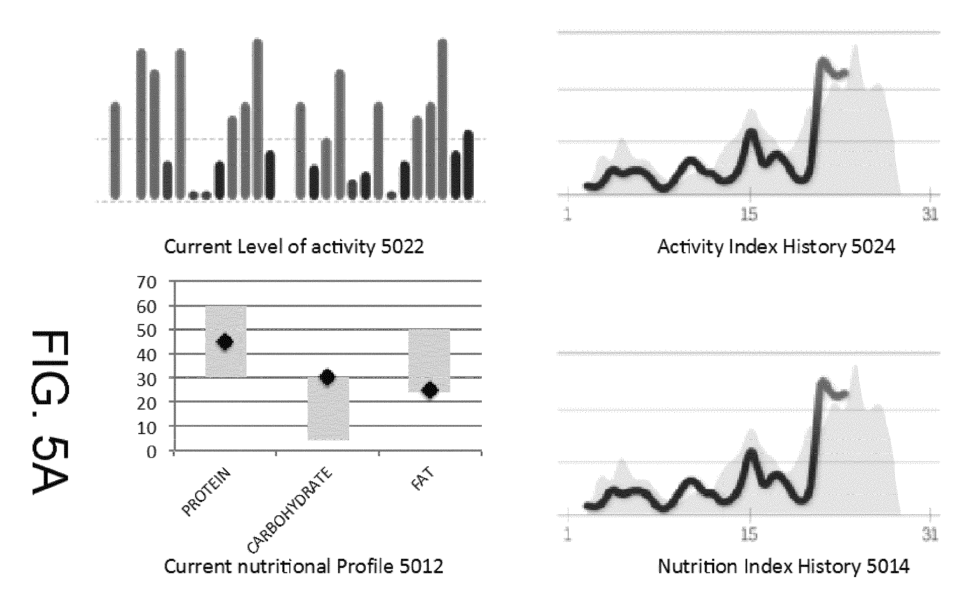

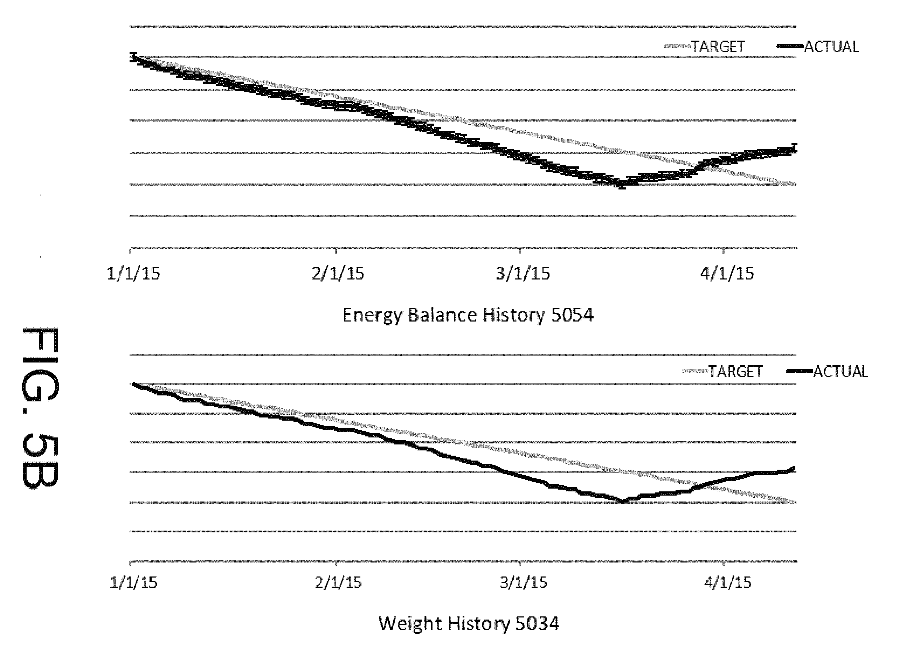

Figure 5a and 5b generally illustrates an embodiment of a website hosted by the webserver 552 comprised in the cloud device 5 and accessible by the terminal device 7. The website can display the status 510 of the system 100, as well as processed data like activity/behavior pattern 502, feeding pattern 501, weight pattern 503, physiological pattern 504 when available or any other processed data coming form measurements performed by the system as well as the results of analysis thereof by the cloud device 5.
Database is configured to transmit a command (509) to the feeding device 3 in response to user input into the website.

In an embodiment, the processed data coming from the portable device 2 can comprise current motion and mobility pattern 5020, optimal motion and mobility pattern 5021, current level of activity 5022, optimal level of activity 5023, activity index history 5024, activity profile 5025, cognitive play pattern 5026 as well as physiological pattern 504 like heart beat pattern 5040, temperature pattern 5041, blood oxygen level pattern 5042. The present disclosure is not limited to displaying the current level of activity of the pet 1 and its history but could include any analysis performed of the activity and motion measurements 201 such as specific behavior identification and/or comparison versus similar pet. For example, the level of activity can be represented with a bar chart of different color to indicate the intensity of the activity, the duration of the activity as well as the recommend average.

In an embodiment, the processed data coming from the feeding device 3 can comprise food consumption pattern (frequency, type and calorie) 5010, water consumption pattern 5011, current nutritional profile 5012, optimal nutritional profile 5013, nutrition index history pattern 5014; The current nutritional profile 5012 can show what have been eaten while the optimal profile 5013 can be in form of a snapshot or aggregated data from given time. The current nutritional profile 5012 can be displayed with the evolution time of nutrition index history pattern 5014 of the pet 1.

The could database 5 analysis engine can help the pet owner 6 to adjust either the food type, the feeding pattern 501 and/or quantity to help the pet 1 get closer to his individual nutritional profile requirement. For example, the nutritional profile 5012 can be represented with a triangular chart (showing the % of Fat, Protein and Carbohydrate) where the pet optimal profile 5013 is marked with a star and the current nutritional profile 5012 with a circle. In an embodiment, by combining activity/behavior pattern 502, feeding pattern 501 and/or weight pattern 503 and/or physiological pattern 504, the website can enable the pet owner 6 to discover health/wellness evaluation pattern 505 like individual level of energy requirement 5050, optimal energy requirement/ consumption balance 5051, activity/behavior change 5052, current energy balance 5053, energy balance history 5054, water consumption change 5055, food consumption change 5056 of the pet. Those analyses can help predict the pet level of health and wellness and provide recommendation to the pet owner 6 on how to improve the level of health and wellness of his pet.

In an embodiment, the website can enable the owner 6 to define the scope of information and/or alerts that he wants to receive.

In an embodiment, the website can comprise a device status window 555 (not shown). The device status window 555 can indicate whether each of the devices (2,3,4,7) in the system are operational or not. The device status windows 555 can inform the pet owner 6 when software updates including new functionalities or errors correction are available to install.

In another embodiment shown in Figure 1b, the addition of the weight device 4 can enable pet's weight measurements 401 and as a consequence can enable the website to represent the weight pattern 503 like body weight pattern 5029, optimal body weight pattern 5030, excremented feaces pattern 5031, excremented urine pattern 5032 or the evolution versus a predefined corridor define by the pet 1 level of health and wellness.

In a related embodiment, the website can comprise a forum to enable pet owners 6 to share experience amongst each other's and to create a club.

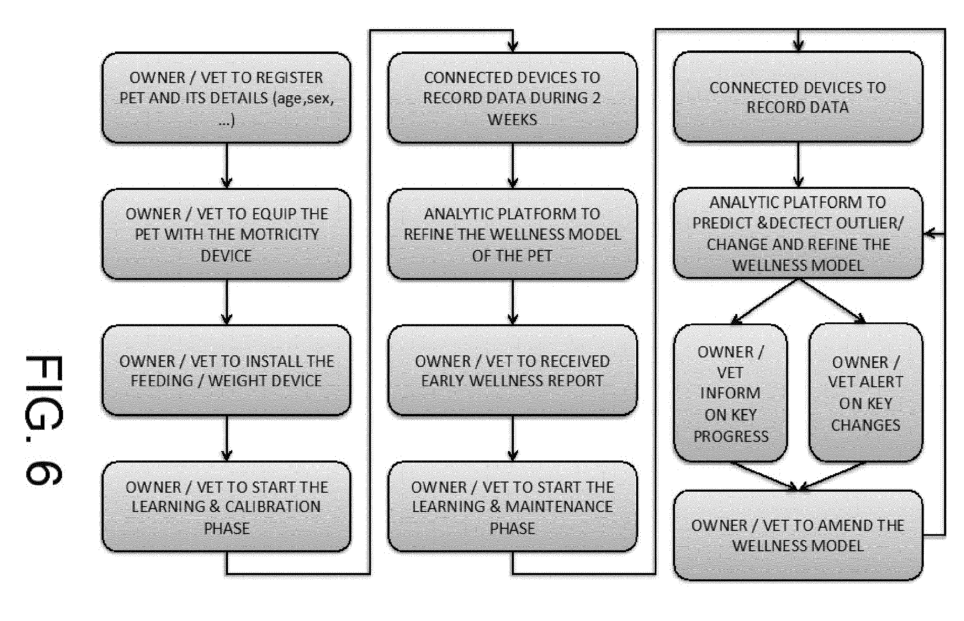

In another aspect, the present disclosure provides a method for pet monitoring which enables receiving information 507 about different factors related to the pet wellness, for example as a single short phrases concerning an individual nutritional requirement or more complex report as illustrated in Figure 6.

In normal use, once the system with all hardware devices is bought it is installed by the Owner. The pet is equipped with a portable device 2 and all other devices are arranged in most suitable places. An external provider governs the cloud device 5 in order to perform the method for pet monitoring.

The method for pet monitoring comprises several steps, some of which are performed repeatedly.

The first step is to provide a cloud database 5 with pet's data like pet species 7010, breed 7011, age 7012, sex 7013, weight 7014, nurtured state 7015,date of birth 7018 and combinations thereof. The system could later be enriched by storing further pet characteristic such as the pet medical history7016, DNA profile 7017. Such data are provided an individual and/or automatic entry through the personal device 7 and/or directly into the cloud database 5 using a specific API.

Additionally, the cloud database 5 will store product' nutrition information and/or scientific pet's data 511 concerning pets such as the major pet medical conditions data 5110 and there impacts on activity level and food consumptions, typical breed's and specie's physiological characteristics per life stage such as typical breed's and specie's weight 5111, typical breed's and specie's level of activity 5112, the typical breed's and specie's food consumptions 5113 and typical breed's and specie's energy requirements 5114. The information could then be modified and/or appended either manually by entry through the personal device 7 and/or directly into the cloud database 5 using a specific API or automatically.

The cloud database 5 will be able to enrich the information by running empirical studies and using those learning in future analysis.

The second step is a step of providing the cloud device 5 with measurements data, in particular with activity and motion measurements 201 and feeding measurements 301. In particular consumption measurements 3010, the amount of food eaten 3011, the amount of water drank 3012, nutrition measurements 3013: product nutrition information 3014, times of filling the bowl 3015 are provided in the second step as feeding measurements 301. In particular distance moved by the pet 2010 and type of activity performed by the pet 2011 are provided in the second step as activity and motion measurements 201.

In another embodiment, the cloud device 5 can be provided not only with the level of motion and activity but as well physiological measurements 211 like real time heart beat 2110, temperature 2111, blood oxygen level 2112 of the pet. Those health parameters will be transferred as well to the database 501 and incorporated in the analysis performed. In another embodiment, the step of providing the cloud device with measurements data also comprises providing weight measurements 401 like the weight of the pet 4010, the weight of the pet excrements (both liquid and/or solid) and/or cognitive play measurements 901.

The third step is a step of performing analysis of at least activity and motion measurements 201 and feeding measurements 301, in particular nutrition measurements 3013, product nutrition information 3014, so as to determine output basic pet characteristics, namely at least activity/behavior pattern 502, feeding pattern 501 which are current motion and mobility pattern 5020, optimal motion and mobility pattern 5021, current level of activity 5022, optimal level of activity 5023,activity index history 5024, activity profile 5025, cognitive play pattern 5026 and food consumption pattern (frequency, type and calorie) 5010, water consumption pattern 5011, current nutritional profile 5012, optimal nutritional profile 5013, nutrition index history pattern 5014.

Additionally the step of performing analysis of input data comprises determination of health/ wellness evaluation pattern 505 based on other patterns described previously. In particular health/ wellness evaluation pattern 505 can include individual level of energy requirement 5050, optimal energy requirement/ consumption balance 5051, activity/behavior change 5052, current energy balance 5053, energy balance history 5054, water consumption change 5055, food consumption change 5056

The fourth step is a step of matching current basic output pet characteristics (patterns) with reference output pet characteristics (patterns) saved in database, so as to determine change and/or deviation from the reference.

In particular the step of matching current output basic pet characteristics with output pet characteristics saved in database comprises matching current nutritional profile 5012 of the food eaten with the optimal nutritional profile 5013 for the pet 1, so as to predict trends and consequences for the pet's wellness and generate reports on changes in feeding behavior.

In addition to the first alternative or as another alternative, the step of matching current basic pet characteristics with pet characteristics saved in database comprises matching the individual level of energy requirement 5050 and the food consumption pattern (frequency, type and calorie) 5010, so as to predict trends and consequences for the pet's wellness and generate reports on changes in feeding behavior.

Advantageously, the method comprises the fifth step which is a step of generating messages to the owner/vet/care taker like the owner/vet/care taker information 507 in form of single phrases or complex reports and transmitting it to the owner device 7. This might be a message to the owner/vet/caretaker about a noticeable fact such as recent visit to the litter box or the bowl, recent clean and/or refill of the littler box or the bowl, recent rest/sleep activity of the pet, recent play activity of the pet, low battery status of a device as well as comparison of the owner animal versus the average population. This step also comprises generating of owner/vet/care taker alert 508 which is a message about change of pattern that might require a visit to the vet such as recent loss/gain of weight, change of drinking/eating pattern, change of activity pattern, change of activity level, change of visit frequency to the litter box. This step also comprises generating feeding recommendation 506.

The method of pet monitoring described in the present disclosure is a process with multiple phases, namely it comprises: calibration phase, maintenance phase and alert and/or adjustment phase:
- The calibration phase comprises learning about the pet and refining his individual profile. This phase might last up to 2 weeks and is finished once the statistical model representing the pet individual profile is able to explain/represent/predict the dataset with sufficient accuracy (adjusted R square and/or cross validation/resampling for example).
- The maintenance phase comprises acquiring repeatedly measurements data and doing full data analysis of said measurement in order to identify pattern change and outliers data, examples of which are illustrated in figure 4. The present disclosure does not limit itself to outlier detection.
- The alert and/or adjustment phase comprises transmitting crucial data to the owner so as he/she receives recommendation, for example on how to improve the current pet nutritional profile through preventive action and so as he/she can track the progress along that goal.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A system for pet monitoring comprising:
- a portable device (2) comprising a data storage module (224) for storing at least an individual pet identifier, an activity and motion sensor (222) and a communication module (226) for sending collected measurements and for informing about the proximity of said individual pet;
- a feeding device (3) comprising a data storage module (334), a weight sensor module (332), at least one feeding and/or drinking bowl (339), a first communication module (336);
- and/or a second communication module (337)
- means for food and/or drink type recognition (333);
- a cloud device (5) comprising a database (551), remotely located relative to the feeding device (3) and portable device (2), for receiving and analyzing at least consumption measurements (3010) and nutrition measurements (3013) including product nutrition information (3014)

2. The system according to claim 1, wherein means for food and/or drink type recognition (333) are part of the feeding device (3);

3. The system according to claim 1 or 2, wherein it further comprises at least one pet owner terminal device (7) configured to communicate with the cloud device (5) for receiving at least a portion of the processed measurements and result of analysis as well as messages.

4. The system according to claim 3, wherein means for food and/or drink type recognition (333) are part of the terminal device (7)

5. The system according to any of preceding claims, wherein the portable device (2) comprises means (223) for measuring physiological data.

6. The system according to any of preceding claims, wherein it further comprises a weight device (4) for measuring at least the weight of an individual pet.

7. The system according to any of preceding claims, wherein the cloud device (5) is configured to process collected measurements and/or perform data analysis and generate messages based on at least activity and motion measurements (201) as well as consumption measurements (3010) and nutrition measurements (3013).

8. The system according to any of preceding claims, wherein the cloud device (5) comprises a webserver (552) hosting a website for providing the pet owner through a terminal device (7) with at least a portion of the processed measurements and result of analysis as well as messages.

9. The system according to any of preceding claims, wherein the database (551) stores at least one among the following: product nutrition information, scientific pet's data (511) related to the major pet medical conditions data (5110) and their impact on the pet weight, typical breed's and specie's physiological characteristics per life stage such as typical breed's and specie's weight (5111), typical breed's and specie's level of activity (5112), typical breed's and specie's food consumptions (5113) and typical breed's and specie's energy requirements (5114), level of activity, consumption patterns, pet individual physiological characteristic provided by the owner;

10. A method of pet monitoring comprising:
- a step of providing the cloud device (5) with measurements data being at least activity and motion measurements (201), consumption measurements (3010) including the amount of food eaten (3011), the amount of water drank (3012) and nutrition measurements (3013) including product nutrition information (3014);
- a step of performing analysis of at least activity and motion measurements (201), feeding measurements (301) including the amount of food eaten (3011), the amount of water drank (3012) and nutrition measurements (3013) including product nutrition information (3014) so as to determine output pet characteristics including feeding pattern (501), activity/behavior pattern (502) and health/ wellness evaluation pattern (505) including at least individual level of energy requirement (5050);
- a step of matching current output pet characteristics with output pet characteristics saved in database (551), so as to determine change and/or deviation in pet characteristics;

11. The method according to claim 10, wherein it further comprises a step of generating messages to an owner/vet/care taker and transmitting it to an pet owner terminal device (7).

12. The method according to claim 10 or 11, wherein it further comprises a step of providing the cloud device (5) with pet's data (701) comprising at least one among species (7010), breed (7011), age (7012), sex (7013), weight (7014), nurtured state (7015), medical history (7016), DNA profile (7017), date of birth (7018) and/or scientific pet's data (511) comprising at least one among the major pet medical conditions data (5110) and their impact on the pet weight, typical breed's and specie's physiological characteristics per life stage such as typical breed's and specie's weight (5111), typical breed's ,specie's level of activity (5112), typical breed's and specie's food consumptions (5113), typical breed's and specie's energy requirements (5114) and storing in the cloud database (551).

13. The method according to claim 10 or 11 or 12, wherein weight measurements (401) like the weight of the pet (4010), the weight of the pet excrements (4011) are provided in the step of providing the cloud device (5) with measurements data.
